Europäisches Patentamt

(19) European Patent Office

Office européen des brevets

(11) EP 0 737 926 A1

## (12) EUROPEAN PATENT APPLICATION

(43) Date of publication:
16.10.1996 Bulletin 1996/42

(51) Int. Cl.$^6$: G06F 17/00, A61K 9/00

(21) Application number: 96105625.6

(22) Date of filing: 10.04.1996

(84) Designated Contracting States:
AT BE CH DE DK ES FI FR GB GR IE IT LI LU MC NL PT SE

(30) Priority: 11.04.1995 US 420582

(71) Applicant: HOECHST MARION ROUSSEL, INC.
Somerville, New Jersey 08876-1258 (US)

(72) Inventor: Natarajan, Chandra
Plainsboro, New Jersey 08536 (US)

(74) Representative: Losert, Wolfgang Dr.
Hoechst AG
Patent- und Lizenzabteilung
Gebäude K 801
D-65926 Frankfurt am Main (DE)

(54) **Method for determining pharmacodynamic parameters and designing a dosing regimen for a drug**

(57) Method for determining a particular characteristic value of an effect compartment, such as the exit rate constant, after administration of a drug to a central compartment coupled to the effect compartment. Drug concentrations in the effect compartment are ascertained as a function of the exit rate constant, and a measured effect of the drug is obtained. A hysteresis loop between the drug concentrations in the effect compartment and the measured effect is graphically formed, and the hysteresis loop is collapsed by varying the exit rate constant of the effect compartment. A pharmacodynamic model which fits the curve of the collapsed hysteresis loop is selected, and a predicted effect is obtained in accordance with the selected pharmacodynamic model and the drug concentrations in the effect compartment. An error between the predicted effect and the measured effect is calculated, and the exit rate constant of the effect compartment is then adjusted until the calculated error between the predicted effect and the measured effect is minimized, thus obtaining the exit rate constant of the effect compartment. A dosing regimen for the drug is then designed in accordance with the exit rate constant at which the calculated error is minimized.

**Description**

## BACKGROUND OF THE INVENTION

The present invention relates to a method for determining pharmacodynamic parameters and a dosing regimen for a drug and, more particularly, to a method for determining the exit rate constant of an effect compartment upon administration of a drug to a central compartment coupled to the effect compartment and designing a dozing regimen for a drug utilizing the determined exit rate constant.

Drug effect is a function of both dosage and time, and it is a major objective of clinical pharmacology to understand this relationship. Dose-effect data are typically depicted by plotting the dose as the independent variable and the measured effect as the dependent variable. From the resulting dose-effect curve, the maximal efficacy, or dosage at which no practical increased therapeutic effect will occur, can be determined.

To achieve a therapeutic objective by administering a drug, a pharmacologic response must be attained and maintained. This in turn requires an appropriate concentration of drug at the site of action. The field of pharmacokinetics involves the study of the time course of a drug's concentration in the body. Drug concentrations may be predicted at the site of measurement (e.g. plasma, urine, or saliva), or in hypothetical effect "compartments," which cannot be sampled directly, wherein the effect site is modelled as a compartment linked to a pharmacokinetic compartment. Concentrations are usually described as a function of both dose and time.

The pharmacokinetic behavior of most drugs is a function of certain parameters, which include the speed of absorption (represented by the absorption rate constant $K_a$), the extent of drug absorption (bioavailability, or F), dose (D), volume of distribution ($V_d$), fraction of unbound drug, plasma drug concentration ($C_p$), clearance rates (renal and extrarenal, i.e. metabolic), the elimination rate constant (K) and biologic half life (the time required for the plasma drug concentration or the amount in the body to decrease by 50%).

Pharmacokinetic parameters are constants, although their values may differ from one patient to another and in the same patient under different conditions. Variables affecting pharmacokinetic parameters include the age and weight of the patient; renal function impairment, which will affect renal clearance; hepatic disease, which will affect metabolic clearance; other diseases such as heart failure, pneumonia, and hyperthyroidism; interactions with other drugs; and method of administration. The values of pharmacokinetic parameters are determined experimentally and once determined, the kinetics of a drug can be predicted.

The traditional method of drug therapy involves empirically adjusting dosage until the therapeutic objective is met. However, this method is unsatisfactory because the dosage may be increased prematurely (with possible toxicity to the patient) during the lag between the time the drug is administered and the time when its effects are seen. Adjusting dosage by monitoring the plasma concentration of the drug is not an ideal approach either because plasma concentration is not necessarily an adequate reflection of the concentration of the drug in the immediate environment where its effect is intended. Due to the multi-exponential nature of the plasma concentration time course, it cannot generally be assumed that it will parallel the time course of effect-site concentration.

Rational dosing regimens cannot adequately be designed with a knowledge of pharmacokinetics alone. Knowledge of the time course of drug concentration cannot alone predict the time course or magnitude of a drug's effect. A knowledge of a drug's pharmacodynamics is also required. Pharmacodynamics involves the study of the concentration-effect relationship when drug concentrations at the effect site have reached equilibrium and the body's response is constant. However, the application of pharmacodynamics to the study of drug action *in vivo* is limited by the fact that the concentration at the effect site, or $C_e$, cannot easily be measured directly.

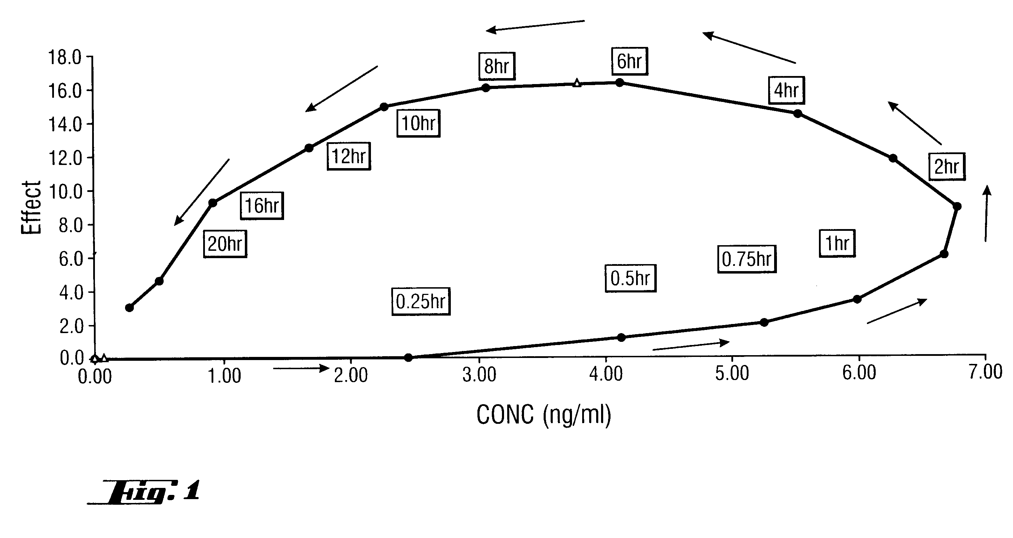

Plasma concentration ($C_p$), which may be measured more easily, may be plotted against effect (E) to estimate the pharmacodynamics of a given drug. However, such a plot generally results in an anticlockwise hysteresis loop representing the equilibration delay (see Fig. 1). To correct for hysteresis, the effect site can be mathematically modelled as a hypothetical compartment linked to one or more of the pharmacokinetic compartments (such as a "plasma" compartment, whose concentration is $C_p$), so that a predicted $C_e$ can be determined and plotted against the observed E to model the pharmacodynamic relationship.

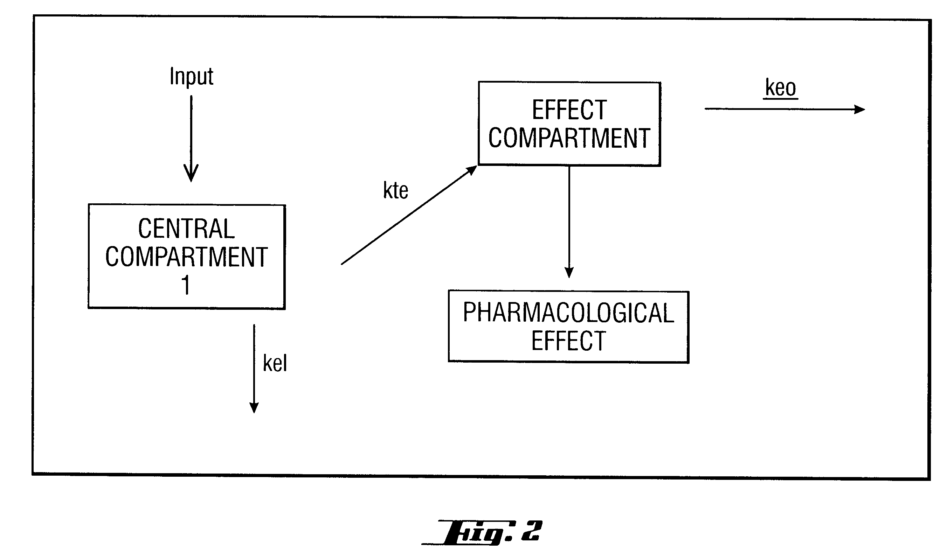

It has been shown that $C_e$, and therefore, effect, is controlled by the exit rate constant for the effect site, $K_{eo}$, and very little by the rate constant $K_{ie}$ which represents the drug movement from the central compartment into the effect compartment [Holford et al., Clinical Pharmacokinetics 6:429-453 (1981)]. Thus, if $K_{eo}$ is known, $C_e$ can be calculated. These relationships are shown in Fig. 2.

To date, the available methods of modelling pharmacodynamics are mathematically intensive, complicated and may be computationally time-consuming.

## OBJECTS OF THE INVENTION

Therefore, it is an object of the present invention to provide a method for determining the exit rate constant of an effect compartment which is not unduly complicated.

Another object of the present invention is to provide a visually interactive method for determining the exit rate constant easily by using the functions of a spreadsheet computer program.

A further object of the present invention is to provide a method for determining the exit rate constant and other parameters using graphical representations.

An additional object of this invention is to provide a method for designing a pharmaceutical dosing regimen for a pharmaceutical which is not unduly complicated.

Various other objects, advantages and features of the present invention will become readily apparent to those of ordinary skill in the art, and the novel features will be particularly pointed out in the appended claims.

## SUMMARY OF THE INVENTION

In accordance with one embodiment of the present invention, a method for determining a particular characteristic value of an effect compartment after administration of a drug to a central compartment involves collapsing the hysteresis loop formed by plotting drug concentrations in the effect compartment, which concentrations are determined in accordance with the characteristic value, and the measured effect of the drug, by varying the characteristic value; selecting a pharmacodynamic model (e.g., $E_{max}$, Sigmoid $E_{max}$, linear and log-linear) which fits the curve of the collapsed hysteresis loop; obtaining a predicted effect of the drug in accordance with the selected pharmacodynamic model and the drug concentrations in the effect compartment; and determining the characteristic value of the effect compartment that minimizes the error between the predicted effect and the measured effect.

As one aspect of the present invention, the hysteresis loop is collapsed by first plotting a curve of the effect compartment concentration with respect to the measured effect, and then collapsing the plotted curve (i.e., causing the ascending limb or limbs of the curve to overlap the descending limb or limbs) by varying the characteristic value.

As a further aspect of the present invention, the characteristic value is determined by adjusting the characteristic value and by adjusting another pharmacodynamic parameter (e.g., the maximum effect attributable to the drug) of the selected pharmacodynamic model so as to minimize the error between the predicted effect and the measured effect.

As another aspect of the present invention, the error between the predicted effect and the measured effect represents the sum of the squares of the differences between the predicted effect and the measured effect.

In still a further aspect of the present invention, the characteristic value is adjusted by utilizing a spreadsheet computer program (e.g., by utilizing the Gauss-Newton search of the Solver function in Microsoft® Excel™) to minimize the error between the predicted effect and the measured effect.

In still another aspect of the present invention, the characteristic value of the effect compartment represents the rate at which the drug exits the effect compartment.

In accordance with another embodiment of the present invention, a method for determining the exit rate constant of an effect compartment involves ascertaining drug concentrations in the effect compartment as a function of the exit rate constant; receiving a measured effect of the drug; graphically forming a hysteresis loop between the drug concentrations in the effect compartment and the measured effect (i.e., by plotting the curve of the drug concentrations in the effect compartment with respect to the measured effect); collapsing the hysteresis loop (i.e., by causing the ascending limb or limbs of the curve to overlap the descending limb or limbs) by varying the exit rate constant of the effect compartment; selecting a pharmacodynamic model (e.g., $E_{max}$, Sigmoid $E_{max}$, linear and log-linear) which fits the curve of the collapsed hysteresis loop; obtaining a predicted effect in accordance with the selected pharmacodynamic model and the drug concentrations in the effect compartment; calculating an error between the predicted effect and the measured effect (e.g., the sum of the squares of the differences between the predicted effect and the measured effect); and adjusting the varied exit rate constant of the effect compartment so as to minimize the calculated error between the predicted effect and the measured effect thereby obtaining the exit rate constant of the effect compartment.

As another aspect of the present invention, the measured effect of the drug is obtained by measuring the effect of the drug at the effect compartment.

As a further aspect of this invention, the calculated error between the predicted effect and the measured effect is minimized by adjusting the varied exit rate constant of the effect compartment and by adjusting another pharmacodynamic parameter (e.g., the maximum effect attributable to the drug).

As another aspect of the present invention, the varied exit rate constant is adjusted by utilizing a spreadsheet computer program (e.g., by utilizing the Solver function in Microsoft® Excel™) to minimize the error between the predicted effect and the measured effect.

In accordance with a further embodiment of the present invention, the method for designing a dosing regimen for a drug involves receiving pharmacokinetic parameters for the drug; ascertaining drug concentrations in an effect compartment in accordance with the pharmacokinetic parameters as a function of the exit rate constant of the drug from the effect compartment; receiving a measured effect of the drug; graphically forming a hysteresis loop between the drug concentrations in the effect compartment and the measured effect; collapsing the hysteresis loop by varying the exit rate constant of the effect compartment; selecting a pharmacodynamic model (e.g., $E_{max}$, sigmoid $E_{max}$, linear and log-linear) which fits a curve of the collapsed hysteresis loop; obtaining a predicted effect in accordance with the selected pharmacodynamic model and the drug concentrations in the effect compartment; calculating an error between the predicted effect and the measured effect (e.g., the sum of the squares of the differences between the predicted effect and the measured effect); and adjusting the varied exit rate constant of the effect compartment (e.g., by using the capabilities of a spreadsheet program) so as to minimize the calculated error between the predicted effect and the measured effect; and designing the dosing regimen for the drug in accordance with the exit rate constant at which the calculated error is minimized.

As one aspect of the present invention, a dose size and a dosing interval for the drug are determined which will maintain the ideal drug concentration at the effect site so as to produce a desired therapeutic response.

As a further aspect of this invention, the drug concentrations in the effect compartment are ascertained from the absorption rate of the drug, the dosage of the drug, and the volume of distribution of the central compartment which is coupled to the effect compartment.

## BRIEF DESCRIPTION OF THE DRAWINGS

The following detailed description, given by way of example and not intended to limit the present invention, will be appreciated best in conjunction with the accompanying drawings, wherein like reference numerals denote like elements and parts, in which:

Fig. 1 is a schematic diagram of plasma concentration versus effect resulting in a hysteresis loop;
Fig. 2 is a schematic illustration of drug movement into and out of various compartments;
Fig. 3 is a schematic diagram illustrating the collapsed hysteresis loop;
Figs. 4A and 4B schematically illustrate the "visible spreadsheet" of various parameters and plots, respectively;
Fig. 5 is a schematic plot of the $C_e$-E relationship in the $E_{max}$ pharmacodynamic model;
Figs. 6A-6C schematically illustrate the linear pharmacodynamic model;
Fig. 7 schematically illustrates the log-linear pharmacodynamic model;
Fig. 8 schematically illustrates the sigmoid $E_{max}$ pharmacodynamic model;
Fig. 9 is a schematic plot of an exemplary $C_e$-E relationship that matches the sigmoid $E_{max}$ pharmacodynamic model; and
Fig. 10 is an exemplary spreadsheet showing various pharmacokinetic and pharmacodynamic parameters.

## DETAILED DESCRIPTION OF CERTAIN PREFERRED EMBODIMENTS

Using modern spreadsheet computer programs, e.g., Microsoft® Excel™, the concentration of a drug in a central compartment may easily be plotted against the observed effect E to produce an anticlockwise hysteresis loop, as illustrated in Fig. 1 of the drawings. Although the central compartment may represent plasma, saliva, urine, or other sampled sites, for purposes of this discussion, the central compartment represents plasma, however, such should not be construed as a limitation on this invention.

The plasma concentration $C_p$ varies as a function of time and can be calculated using various pharmacokinetic parameters including dosage D, bioavailability F, central compartment volume of distribution $V_d$, the absorption rate constant $K_a$, and various rate constants, as is well known in the art. For example, Holford et al. "Understanding the Dose-Effect Relationship: Clinical Application of Pharmacokinetic-Pharmacodynamic Models," Clinical Pharmacodynamics 6, 429-53 (1981), which is incorporated herein by reference, provides descriptions of various pharmacokinetic models, as well as various pharmacodynamic models.

Equation 1 represents one particular pharmacokinetic model.

$$C_P = \left( \frac{F \cdot D}{V_d} \right) \left( \frac{K_a}{K_a - K} \right) \left( e^{-K \cdot t} - e^{-K_a \cdot t} \right) \qquad (1)$$

The effect site concentration $C_e$ is a function of $C_p$ and the exit rate constant $K_{eo}$ only, and therefore, an initial plot of $C_e$ versus E (hereinafter $C_e$-E) is generated by either setting $K_{eo}$ to an arbitrary value or to a value that causes $C_p$ to equal $C_e$. Equation 2 provides the value of $C_e$ as a function of various pharmacokinetic parameters, listed above, and $K_{eo}$ in a one compartment first order absorption pharmacokinetic model.

$$C_e = \frac{D \cdot K_a \cdot K_{eo}}{V_d} \left[ \frac{e^{-KT}}{(K_a - K)(K_{eo} - K)} + \frac{e^{-K_a T}}{(K - K_a)(K_{eo} - K_a)} + \frac{e^{-K_{eo} T}}{(K - K_{eo})(K_a - K_{eo})} \right] \quad (2)$$

Using equation 2, or other known equations representing other pharmacokinetic models, the plot of $C_e$-E generally results in an anticlockwise hysteresis loop (although this may not be the case since $K_{eo}$ is arbitrarily selected). It is appreciated that when $K_{eo}$ is set such that $C_p$ equals or is approximately equal to $C_e$, the $C_e$-E curve is superimposed over the $C_p$-E curve.

Other pharmacokinetic models include one compartment bolus, two compartment bolus, three compartment bolus, two compartment first order absorption, three compartment first order absorption, one compartment zero order absorption, two compartment zero order absorption, three compartment zero order absorption, as well as other pharmacokinetic models known in the art. For purposes of brevity, these models are not discussed herein except where necessary for a clear understanding of the present invention. Similarly, the equations describing the effect-site concentration for these models are well known in the art and thus are not provided here.

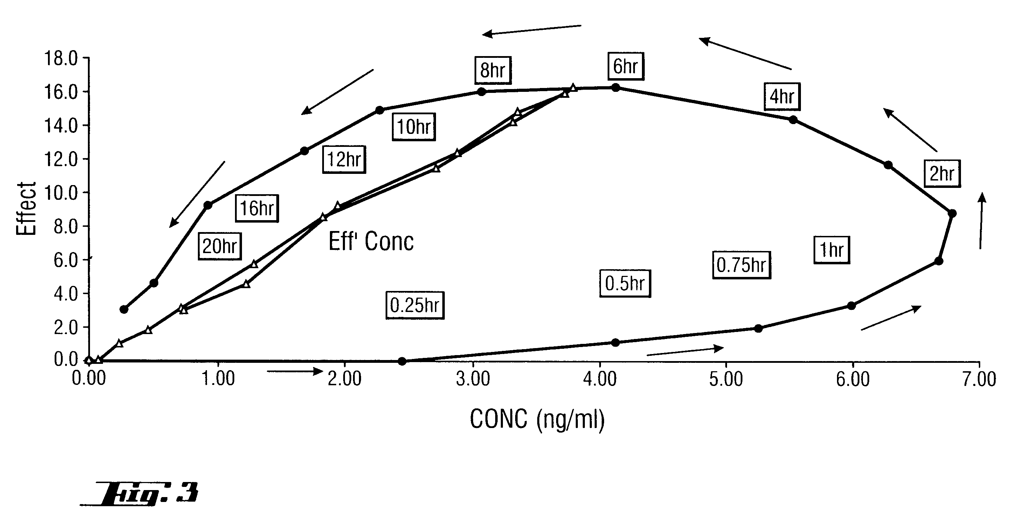

In accordance with the present invention, an initial estimate of $K_{eo}$ is established by collapsing the $C_e$-E hysteresis loop. This is accomplished by varying the value of $K_{eo}$ such that the ascending and descending limbs of the loop approach one another until they overlap. Fig. 3 is a schematic diagram illustrating the hysteresis loop of Fig. 1, as well as the same loop collapsed by varying $K_{eo}$. when the $C_e$-E curve contains more than one hysteresis loop, a $K_{eo}$ value is chosen which results in all of the limbs of the curve being superimposed.

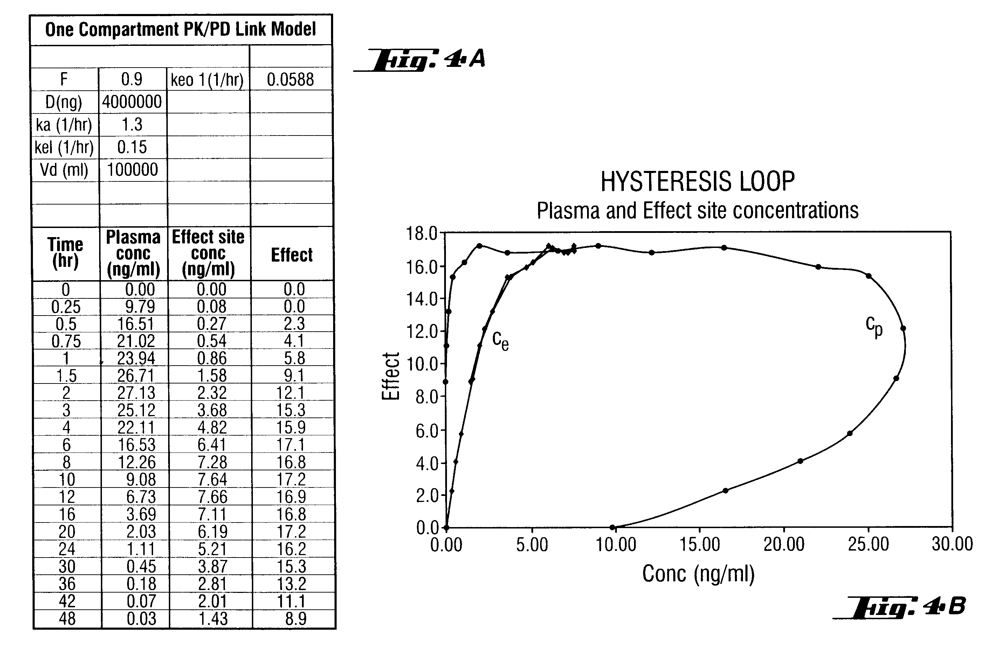

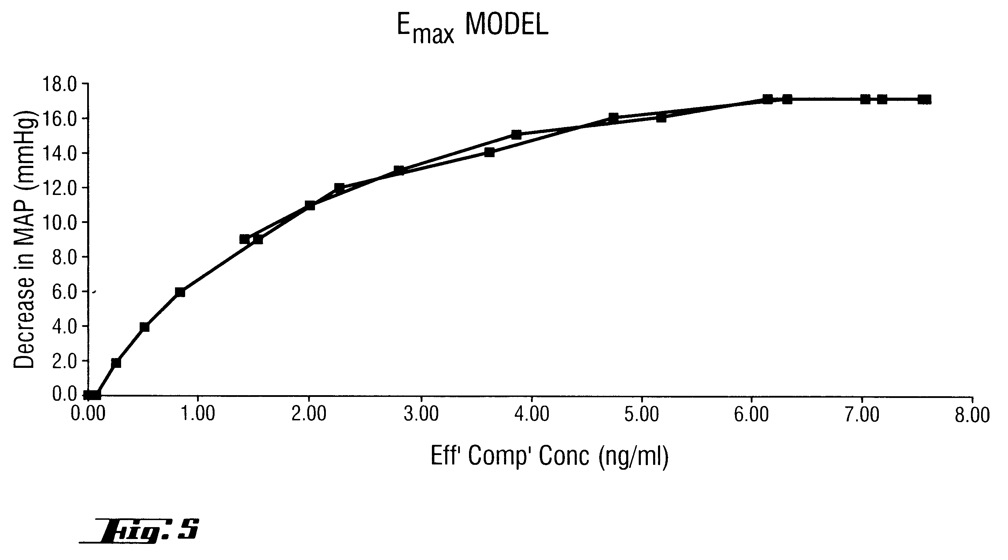

In a preferred embodiment of the present invention, the process of collapsing the hysteresis loop is accomplished using a spreadsheet program, such as Microsoft® Excel™, whereby $C_e$, the values of which are a function of $C_p$ and $K_{eo}$ (where the value of $C_p$ varies with time), is plotted against the observed effect E, the value of which changes with time. In such a spreadsheet program, particular "cells" represent different constants/variables, e.g., $K_{eo}$, dose D, $K_a$, etc, depending on the pharmacokinetic model. The cell designated as the "$K_{eo}$" is varied, as previously described, thereby changing the displayed $C_e$-E plot. Since this plot is "visible" to the user, the value of $K_{eo}$ is easily varied until the hysteresis loop collapses (see Fig. 3). Fig. 4A illustrates one example of the "visible spreadsheet" of the values of $C_e$, E and $K_{eo}$ (and also $C_p$ and time), and Fig. 4B illustrates the $C_e$-E plot of these values (along with the $C_p$-E plot) when the hysteresis loop (i.e., the $C_e$-E plot) is collapsed.

In accordance with the present invention, the shape of the collapsed hysteresis loop is fitted (or "matched") to the $C_e$-E curve of the pharmacodynamic model which it most closely resembles, which models are further described below. As is known in the art, there are various pharmacodynamic models which attempt to describe the drug effect over a range of concentrations. These include the $E_{max}$ model, the sigmoid $E_{max}$ model, the linear model, the log-linear model, as well as other models known in the art.

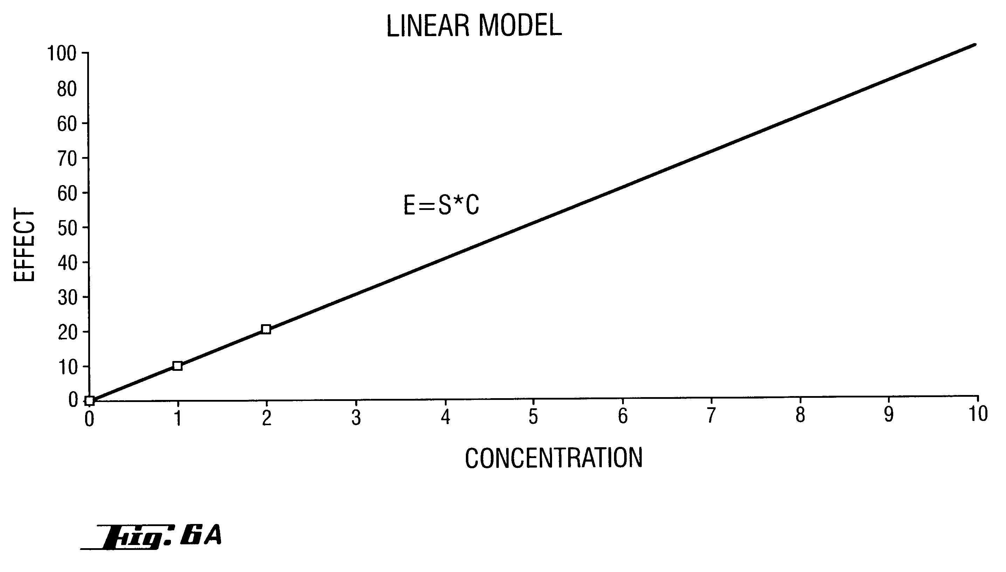

Fig. 5 is a schematic plot of a hypothetical $C_e$-E relationship in the $E_{max}$ pharmacodynamic model. This model adequately describes drug effect over the whole range of concentrations and is based on the hyperbolic relationship of equation (3):

$$E = \frac{E_{max} \cdot C}{EC_{50} + C} \quad (3)$$

Here, $E_{max}$ is the maximum effect attributable to the drug and $EC_{50}$ is the concentration of the drug that produces 50% of $E_{max}$. The $E_{max}$ model incorporates the "law of diminishing returns", reflecting the higher concentrations required to increase the effect by a given amount. As is known in the art, this model may be applied when the drug effect is measured as inhibition of some biological phenomenon, wherein equation (3) can be rewritten as:

$$E = NODRUG - \frac{E_{max} \cdot C}{IC_{50} + C} \quad (4)$$

where NODRUG is the effect when no drug is present and $IC_{50}$ is the concentration of the drug that produces 50% of the maximum inhibition of effect ($E_{max}$). Similarly, variations of the $E_{max}$ model, as well as other models, may also be utilized in the present invention.

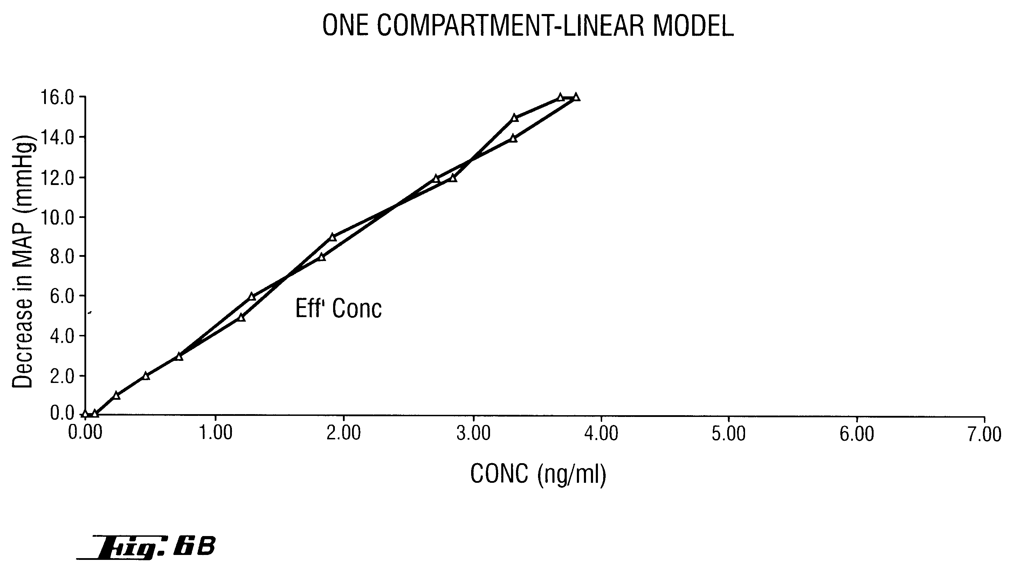

Fig. 6A is a schematic plot of the $C_e$-E relationship in the linear pharmacodynamic model. Generally, if drug concentrations are low in relation to $EC_{50}$, the effect becomes proportional to concentration as represented by equation (5):

$$E = S \cdot C \qquad\qquad (5)$$

As is apparent from Fig. 6A and equation (5), S merely represents the slope of the line comparing $C_e$ with effect. Figs. 6B and 6C illustrate collapsed hysteresis loops fitting the linear pharmacodynamic model wherein Fig. 6B represents a one compartment model and Fig. 6C represents a two compartment model. It is to be noted that although the linear pharmacodynamic model generally is unable to predict the maximum effect of a drug, such may not be necessary when it is not possible to achieve concentrations which achieve effects approaching the maximum.

The log-linear pharmacodynamic model similarly illustrates the $C_e$-E relationship wherein the logarithm of concentration is related to the effect, as represented by equation (6):

$$E = S \cdot \log(C) + I \qquad\qquad (6)$$

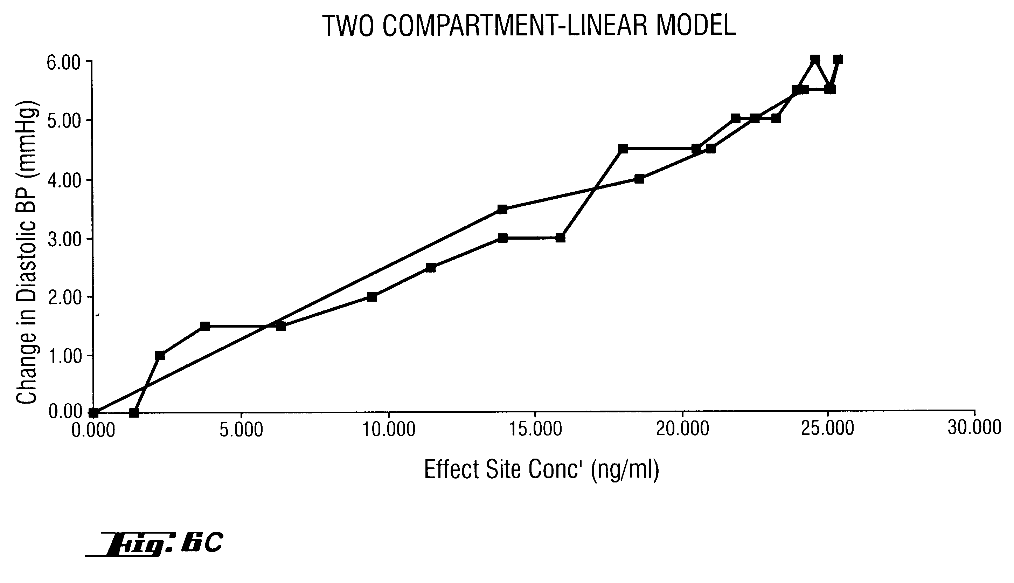

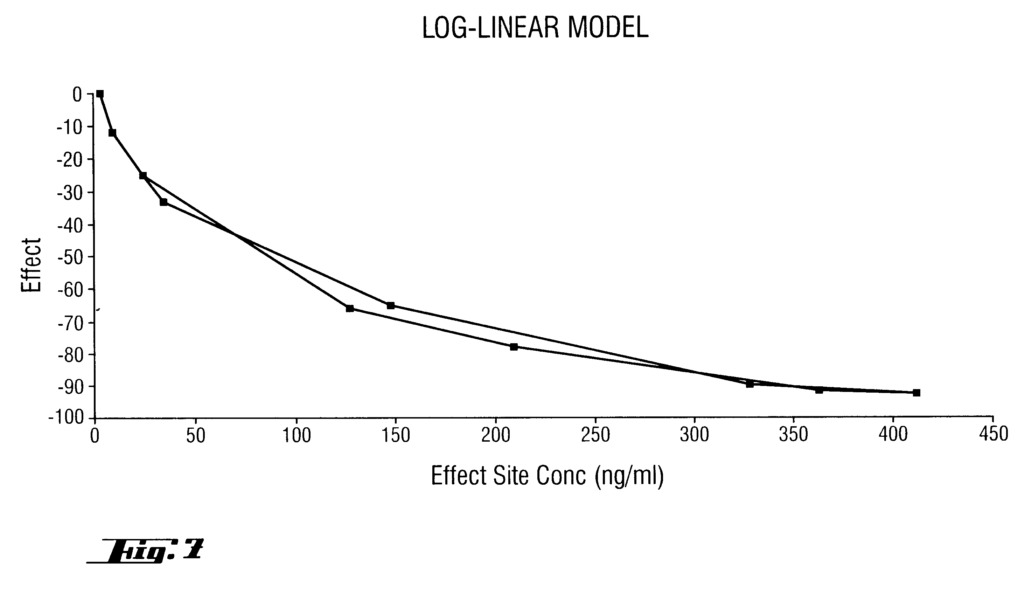

where I is an arbitrary constant with no physical meaning. Fig. 7 is an exemplary plot of the $C_e$-E relationship in the log-linear pharmacodynamic model. However, as is apparent from the figure, there is an increased inhibition of effect as $C_e$ increases.

Another pharmacodynamic model, the sigmoid $E_{max}$ model, although similar to the $E_{max}$ model, describes drug effect again over the whole range of concentrations wherein the C-E curve for a particular drug is not represented simply by the hyperbolic form of the $E_{max}$ model. This model is defined by the equation:

$$E = \frac{E_{max} \cdot C^N}{EC_{50}^N + C^N} \qquad\qquad (7)$$

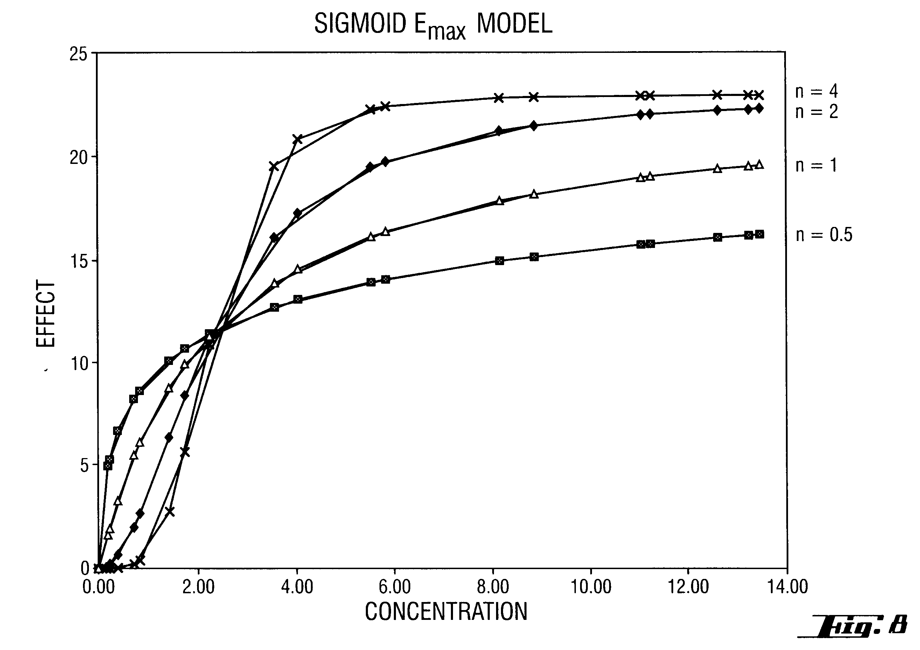

N, here, is a number influencing the slope of the curve, as illustrated in Fig. 8.

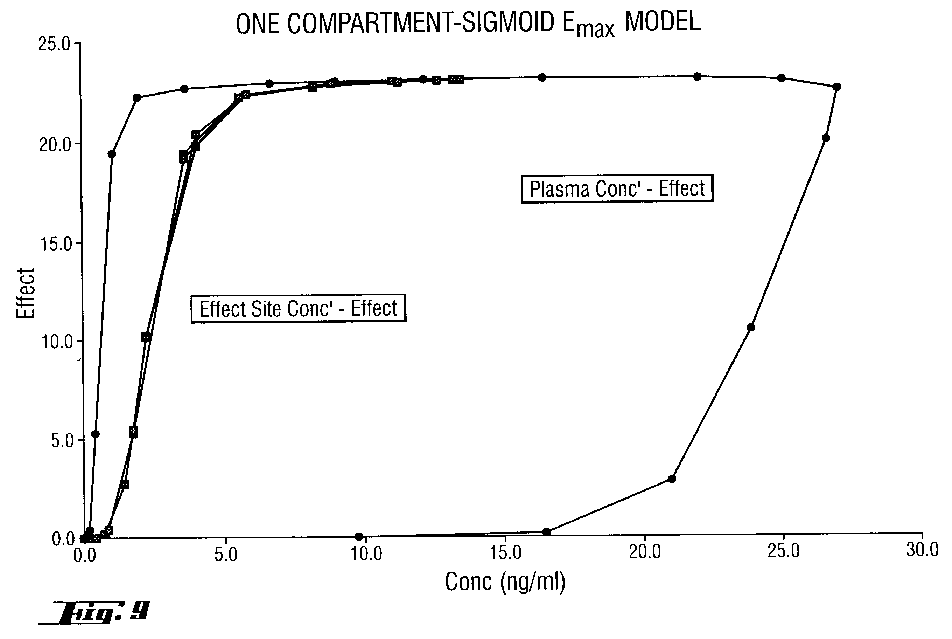

After the concentration-effect hysteresis loop is collapsed in the manner described above, the shape of the collapsed hysteresis loop is evaluated and fitted (or matched) to the shape of the $C_e$-E curve of one of the pharmacodynamic models. For example, the shape of the collapsed hysteresis loop illustrated in Fig. 9 matches the shape of the $C_e$-E relationship shown in the sigmoid $E_{max}$ pharmacodynamic model of Fig. 8, where N (or sigma) is greater than 1. When the pharmacodynamic model is selected based on this "matching" of curves, the selected model is utilized to "fine tune" $K_{eo}$, as further described below. For the above example, the sigmoid $E_{max}$ model, represented by equation (7) above, is selected for the process to be described.

In accordance with the present invention, the selected pharmacodynamic model is utilized to calculate a "predicted" effect $E_p$ of a drug after the administration of that drug with such predicted effect being "fined tuned" as follows:

Using the particular pharmacodynamic model selected and the value of $K_{eo}$ at which the hysteresis loop is collapsed, the predicted effect $E_p$ is calculated from the effect site concentration $C_e$. As previously described, the effect site concentration of a drug is a function of $C_p$ and $K_{eo}$. It is to be noted that other unknown pharmacokinetic parameters which are used in the selected pharmacodynamic model, e.g., $E_{max}$, $EC_{50}$, may be merely approximated at this point in calculating the predicted effect $E_p$.

Then, for each instance in time, the value of $E_p$ is compared to the observed effect E at that particular instance of time, thereby producing an error value which corresponds to the calculated difference between $E_p$ and E. In accordance with this invention, the value of $K_{eo}$ and the values of the other unknown pharmacokinetic parameters are varied until this error is minimized, that is, different values of $K_{eo}$ (and different values of $E_{max}$, $EC_{50}$, etc., for example) are utilized in the selected pharmacodynamic model so that the smallest error between $E_p$ and E may be obtained. In accordance with a preferred embodiment of this invention, an overall error between $E_p$ and E is calculated as follows: First, the differences between each value of $E_p$ (with respect to time) and the corresponding value of the measured effect E at the particular time are calculated. Second, the value of each of the ascertained differences is squared. Finally, the squared differences are summed together to produce the overall error between $E_p$ and E.

To obtain the smallest possible overall error between the predicted effect and the measured effect, different values of $K_{eo}$ (and $E_{max}$, $EC_{50}$, etc, depending on the selected pharmacodynamic model) are utilized in the calculation until the smallest overall error is obtained. The values of $K_{eo}$, $E_{max}$, $EC_{50}$, etc, which produce the smallest error are the "fine tuned" pharmacokinetic models for the particular drug being studied.

In a preferred embodiment of the present invention, this process is performed using a spreadsheet computer program, such as Microsoft® Excel™, in the following manner:

The formulas for known pharmacokinetic models, such as those described herein, are incorporated into the spreadsheet program. Various pharmacokinetic parameters will have been determined during Phase I Clinical trials, wherein a drug is administered to human volunteers for the first time in order to study the metabolism and excretion of the drug and to estimate its potential for producing adverse effects. The appropriate pharmacokinetic model is selected and the values for the pharmacokinetic parameters determined during Phase I Clinical trials are entered in the cells designated for each. A plot of $C_e$ against the observed effect E is generated in the spreadsheet.

The cell designated as "$K_{eo}$" is varied, as previously described, so that the limbs (e.g., ascending and descending) of the displayed $C_e$-E plot converge, until the hysteresis loop collapses (see Fig. 4B). At this point the shape of the curve of the collapsed loop, which is visible on the computer screen to the viewer, is evaluated to determine the most suitable pharmacodynamic model.

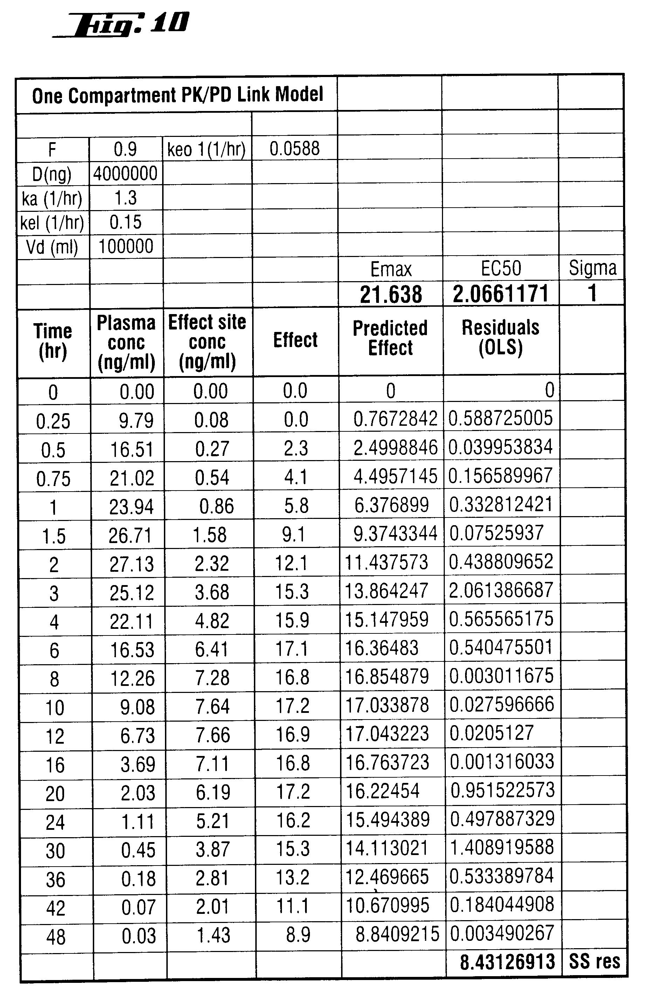

The selected pharmacodynamic model and the value of $K_{eo}$ at which the hysteresis loop is collapsed are then utilized to calculate the predicted effect $E_p$ of the drug from the effect site concentration $C_e$. The squared difference in values between observed E and predicted $E_p$ is calculated for each instance in time. Fig. 10 illustrates an exemplary spreadsheet showing the various parameters (e.g., F, D, $K_a$, $K_{eo}$, $E_{max}$, $EC_{50}$, Sigma), and $C_e$, E, $E_p$ and the error for each instance in time. The overall error (value 8.43126913 in Fig. 10) is calculated by adding all of the errors.

The "Solver" function of Microsoft® Excel™ (or similar functions in other spreadsheet programs, e.g., Lotus-123 or Quattro Pro) is used to "fit" the $C_e$-E curve on the $C_e$-$E_p$ curve by setting the objective of the Solver function to minimize the calculated overall error, that is, to minimize the sum of the squares of all of the differences between E and $E_p$. The Solver is asked to minimize this overall error by changing the values in those cells that correspond to values of $K_{eo}$ and other pharmacokinetic parameters (e.g. $E_{max}$, $EC_{50}$, sigma, etc.). When the overall error is minimized, the current values of $K_{eo}$ and the other pharmacokinetic parameters at that point provide the most accurate estimate of the effect site concentration.

The use of pharmacodynamic models to describe drug effects *in vivo*, in combination with known pharmacokinetic parameters, can be useful in the design of a rational dosing regimen for a drug. For example, upon collapsing the hysteresis loop, if the $C_e$-E curve is best described by an $E_{max}$ model, it would be clear that increasing the dose of a particular drug beyond a given point would be of minimal benefit therapeutically. This saturation of effect may not be readily determined by observing the $C_p$-E curve due to the anticlockwise hysteresis loop. The method of the claimed invention allows the optimal dose size and dosage interval to be determined which will maintain the ideal $C_e$ that produces the desired E. Thus the therapeutic objective is obtained while the risk of toxicity to the patient is reduced.

While the present invention has been particularly shown and described in conjunction with preferred embodiments thereof, it will be readily appreciated by those of ordinary skill in the art that various changes and modifications may be made without departing from the spirit and scope of the invention. For example, the present invention is not limited to any particular pharmacokinetic or pharmacodynamic model described above and may be utilized with any pharmacokinetic model and any pharmacodynamic model known in the art.

As another example, although the present discussion is directed to determining the exit rate constant and other pharmacodynamic parameters, such as $E_{max}$ and $EC_{50}$, the present invention is not limited solely to these parameters and may be widely applied to determining other pharmacodynamic parameters such as $IC_{50}$.

Still further, although the present invention has been described as utilizing Microsoft® Excel™ as the means by which the calculated error between the predicted effect and the measured effect is minimized, other computer programs, as well as manual computational methods, that are available for performing such calculations may be utilized in this invention.

Therefore, it is intended that the appended claims be interpreted as including the embodiments described herein, the alternatives mentioned above, and all equivalents thereto.

**Claims**

1. Method for determining a characteristic value of an effect compartment after administration of a drug to a central compartment coupled to said effect compartment, comprising the steps of:

collapsing a hysteresis loop between drug concentrations in said effect compartment and a measured effect of said drug by varying said characteristic value, said drug concentrations in said effect compartment being determined in accordance with said characteristic value;
selecting a pharmacodynamic model fitting a curve of the collapsed hysteresis loop;
obtaining a predicted effect in accordance with the selected pharmacodynamic model and said drug concentrations in said effect compartment; and

determining the characteristic value of said effect compartment that minimizes an error between said predicted effect and said measured effect.

2. The method of claim 1, further comprising the step of ascertaining drug concentrations in said central compartment in accordance with said characteristic value; and wherein said drug concentrations in said effect compartment are ascertained from said drug concentrations in said central compartment.

3. The method of claim 1, further comprising the step of measuring the effect of said drug at said effect compartment to provide said measured effect.

4. The method of claim 1, wherein the step of collapsing said hysteresis loop is carried out by plotting a curve of said drug concentrations in said effect compartment with respect to said measured effect, and collapsing the curve by varying said characteristic value.

5. The method of claim 4, wherein said curve has an ascending limb and a descending limb; and the curve is collapsed by varying said characteristic value to cause said ascending limb of said curve to overlap said descending limb.

6. The method of claim 4, wherein said curve has a plurality of limbs; and said curve is collapsed by varying said characteristic value to cause each of said limbs to overlap one another.

7. The method of claim 1, wherein a plurality of pharmacodynamic models include $E_{max}$, sigmoid $E_{max}$, linear and log-linear models.

8. The method of claim 1, wherein the selected pharmacodynamic model includes a plurality of parameters; and said predicted effect is obtained from said parameters of said selected pharmacodynamic model and said drug concentrations in said effect compartment.

9. The method of claim 8, wherein the step of determining the characteristic value is carried out by adjusting said characteristic value of said effect compartment and adjusting at least one of said parameters of said selected pharmacodynamic model to minimize the error between said predicted effect and said measured effect.

10. The method of claim 9, wherein said at least one of said parameters is a maximum effect attributable to said drug.

11. The method of claim 1, wherein the error between said predicted effect and said measured effect represents a sum of squares of differences between said predicted effect and said measured effect.

12. The method of claim 1, wherein said characteristic value is adjusted utilizing a spreadsheet computer program to minimize the error between said predicted effect and said measured effect.

13. The method of claim 12, wherein said spreadsheet computer program is Microsoft® Excel™; and wherein said step of determining the characteristic value is carried out by executing Microsoft® Excel™'s Solver function to determine the value of the characteristic value when the error between said predicted effect and said measured effect is minimized.

14. The method of claim 1, wherein said characteristic value of said effect compartment represents a rate at which said drug exits said effect compartment.

15. Method for determining the exit rate constant of an effect compartment after administration of a drug to a central compartment coupled to said effect compartment, comprising the steps of:

> ascertaining drug concentrations in said effect compartment as a function of said exit rate constant;
> receiving a measured effect of said drug;
> graphically forming a hysteresis loop between said drug concentrations in said effect compartment and said measured effect;
> collapsing said hysteresis loop by varying said exit rate constant of said effect compartment;
> selecting a pharmacodynamic model fitting a curve of the collapsed hysteresis loop;
> obtaining a predicted effect in accordance with the selected pharmacodynamic model and said drug concentrations in said effect compartment;

calculating an error between said predicted effect and said measured effect; and

adjusting the varied exit rate constant of said effect compartment to minimize the calculated error between said predicted effect and said measured effect so as to obtain the exit rate constant of said effect compartment.

16. The method of claim 15, further comprising the step of ascertaining drug concentrations in said central compartment in accordance with said exit rate constant; and wherein said drug concentrations in said effect compartment are ascertained from said drug concentrations in said central compartment.

17. The method of claim 15, wherein said step of receiving a measured effect of said drug includes the step of measuring the effect of said drug at said effect compartment.

18. The method of claim 15, wherein the step of graphically forming a hysteresis loop is carried out by plotting the curve of said drug concentrations in said effect compartment with respect to said measured effect.

19. The method of claim 18, wherein said curve has an ascending limb and a descending limb; and said step of collapsing said hysteresis loop is carried out by varying said exit rate constant to cause said ascending limb of said curve to overlap said descending limb.

20. The method of claim 18, wherein said curve has a plurality of limbs; and said step of collapsing said hysteresis loop is carried out by varying said exit rate constant to cause each of said limbs to overlap one another.

21. The method of claim 15, wherein a plurality of pharmacodynamic models include $E_{max}$, sigmoid $E_{max}$, linear and log-linear models.

22. The method of claim 15, wherein the selected pharmacodynamic model includes a plurality of parameters; and said predicted effect is obtained from said parameters of said selected pharmacodynamic model and said drug concentrations in said effect compartment.

23. The method of claim 22, wherein the calculated error between said predicted effect and said measured effect is minimized by adjusting the varied exit rate constant of said effect compartment and adjusting at least one of said parameters of said selected pharmacodynamic model.

24. The method of claim 23, wherein said at least one of said parameters is a maximum effect attributable to said drug.

25. The method of claim 15, wherein said calculated error represents a sum of squares of differences between said predicted effect and said measured effect.

26. The method of claim 15, wherein the varied exit rate constant is adjusted utilizing a spreadsheet computer program to minimize the calculated error between said predicted effect and said measured effect.

27. The method of claim 26, wherein said spreadsheet computer program is Microsoft® Excel™; and wherein said step of adjusting the varied exit rate constant is carried out by executing Microsoft® Excel™'s Solver function to determine the value of the exit rate constant when the calculated error between said predicted effect and said measured effect is minimized.

28. The method of claim 15, wherein said exit rate constant of said effect compartment represents a rate at which said drug exits said effect compartment.

29. Method for designing a dosing regimen for a drug, comprising the steps of:

receiving pharmacokinetic parameters for said drug;
ascertaining drug concentrations in an effect compartment in accordance with said pharmacokinetic parameters as a function of an exit rate constant of said drug from said effect compartment;
receiving a measured effect of said drug;
graphically forming a hysteresis loop between said drug concentrations in said effect compartment and said measured effect;
collapsing said hysteresis loop by varying said exit rate constant of said effect compartment;
selecting a pharmacodynamic model fitting a curve of the collapsed hysteresis loop;

obtaining a predicted effect in accordance with the selected pharmacodynamic model and said drug concentrations in said effect compartment;

calculating an error between said predicted effect and said measured effect; and

adjusting the varied exit rate constant of said effect compartment to minimize the calculated error between said predicted effect and said measured effect; and

designing said dosing regimen for said drug in accordance with the exit rate constant at which the calculated error is minimized.

30. The method of claim 29, wherein said step of designing said dosing regimen includes determining a dose size and a dosing interval which will maintain the drug concentration at the effect site so as to produce a desired therapeutic response.

31. The method of claim 29, further comprising the step of ascertaining drug concentrations in a central compartment coupled to said effect compartment in accordance with said exit rate constant; and wherein said drug concentrations in said effect compartment are ascertained from said drug concentrations in said central compartment.

32. The method of claim 29, wherein said step of receiving a measured effect of said drug includes the step of measuring the effect of said drug at said effect compartment.

33. The method of claim 29, wherein the step of graphically forming a hysteresis loop is carried out by plotting the curve of said drug concentrations in said effect compartment with respect to said measured effect.

34. The method of claim 33, wherein said curve has an ascending limb and a descending limb; and said step of collapsing said hysteresis loop is carried out by varying said exit rate constant to cause said ascending limb of said curve to overlap said descending limb.

35. The method of claim 33, wherein said curve has a plurality of limbs; and said step of collapsing said hysteresis loop is carried out by varying said exit rate constant to cause each of said limbs to overlap one another.

36. The method of claim 29, wherein a plurality of pharmacodynamic models include $E_{max}$, Sigmoid $E_{max}$, linear and log-linear models.

37. The method of claim 29, wherein the selected pharmacodynamic model includes a plurality of parameters; and said predicted effect is obtained from said parameters of said selected pharmacodynamic model and said drug concentrations in said effect compartment.

38. The method of claim 37, wherein the calculated error between said predicted effect and said measured effect is minimized by adjusting the varied exit rate constant of said effect compartment and adjusting at least one of said parameters of said selected pharmacodynamic model.

39. The method of claim 38, wherein said at least one of said parameters is a maximum effect attributable to said drug.

40. The method of claim 29, wherein said calculated error represents a sum of squares of differences between said predicted effect and said measured effect.

41. The method of claim 29, wherein the varied exit rate constant is adjusted utilizing a spreadsheet computer program to minimize the calculated error between said predicted effect and said measured effect.

42. The method of claim 41, wherein said spreadsheet computer program is Microsoft® Excel™; and wherein said step of adjusting the varied exit rate constant is carried out by executing Microsoft® Excel™'s Solver function to determine the value of the exit rate constant when the calculated error between said predicted effect and said measured effect is minimized.

43. The method of claim 42, wherein said exit rate constant of said effect compartment represents a rate at which said drug exits said effect compartment.

44. The method of claim 29, wherein said pharmacokinetic parameters for said drug include the absorption rate of said drug, a dosage of said drug, and a volume of distribution of a central compartment coupled to said effect compartment.

**45.** The method of claim 44, wherein said drug concentrations in said effect compartment are ascertained from the absorption rate of said drug, the dosage of said drug, and the volume of distribution of the central compartment.

Fig. 1

EP 0 737 926 A1

Fig. 2

Fig. 3

EP 0 737 926 A1

## One Compartment PK/PD Link Model

| F | 0.9 | keo 1(1/hr) | 0.0588 |
|---|---|---|---|
| D(ng) | 4000000 | | |
| ka (1/hr) | 1.3 | | |
| kel (1/hr) | 0.15 | | |
| Vd (ml) | 100000 | | |

| Time (hr) | Plasma conc (ng/ml) | Effect site conc (ng/ml) | Effect |
|---|---|---|---|
| 0 | 0.00 | 0.00 | 0.0 |
| 0.25 | 9.79 | 0.08 | 0.0 |
| 0.5 | 16.51 | 0.27 | 2.3 |
| 0.75 | 21.02 | 0.54 | 4.1 |
| 1 | 23.94 | 0.86 | 5.8 |
| 1.5 | 26.71 | 1.58 | 9.1 |
| 2 | 27.13 | 2.32 | 12.1 |
| 3 | 25.12 | 3.68 | 15.3 |
| 4 | 22.11 | 4.82 | 15.9 |
| 6 | 16.53 | 6.41 | 17.1 |
| 8 | 12.26 | 7.28 | 16.8 |
| 10 | 9.08 | 7.64 | 17.2 |
| 12 | 6.73 | 7.66 | 16.9 |
| 16 | 3.69 | 7.11 | 16.8 |
| 20 | 2.03 | 6.19 | 17.2 |
| 24 | 1.11 | 5.21 | 16.2 |
| 30 | 0.45 | 3.87 | 15.3 |
| 36 | 0.18 | 2.81 | 13.2 |
| 42 | 0.07 | 2.01 | 11.1 |
| 48 | 0.03 | 1.43 | 8.9 |

Fig. 4A

### HYSTERESIS LOOP
Plasma and Effect site concentrations

Fig. 4B

EP 0 737 926 A1

E<sub>max</sub> MODEL

Fig. 5

EP 0 737 926 A1

LINEAR MODEL

EFFECT

$E=S*C$

CONCENTRATION

Fig. 6A

# ONE COMPARTMENT-LINEAR MODEL

*Fig. 6B*

EP 0 737 926 A1

# TWO COMPARTMENT-LINEAR MODEL

Fig. 6C

EP 0 737 926 A1

# LOG-LINEAR MODEL

*Fig. 7*

EP 0 737 926 A1

SIGMOID E$_{max}$ MODEL

EP 0 737 926 A1

Fig. 8

## ONE COMPARTMENT-SIGMOID $E_{max}$ MODEL

Plasma Conc' - Effect

Effect Site Conc' - Effect

Conc (ng/ml)

_Fig. 9_

# Fig. 10

| One Compartment PK/PD Link Model | | | | | | |
|---|---|---|---|---|---|---|
| | | | | | | |
| F | 0.9 | keo 1(1/hr) | 0.0588 | | | |
| D(ng) | 4000000 | | | | | |
| ka (1/hr) | 1.3 | | | | | |
| kel (1/hr) | 0.15 | | | | | |
| Vd (ml) | 100000 | | | | | |
| | | | | Emax | EC50 | Sigma |
| | | | | **21.638** | **2.0661171** | **1** |
| Time (hr) | Plasma conc (ng/ml) | Effect site conc (ng/ml) | Effect | Predicted Effect | Residuals (OLS) | |
| 0 | 0.00 | 0.00 | 0.0 | 0 | 0 | |
| 0.25 | 9.79 | 0.08 | 0.0 | 0.7672842 | 0.588725005 | |
| 0.5 | 16.51 | 0.27 | 2.3 | 2.4998846 | 0.039953834 | |
| 0.75 | 21.02 | 0.54 | 4.1 | 4.4957145 | 0.156589967 | |
| 1 | 23.94 | 0.86 | 5.8 | 6.376899 | 0.332812421 | |
| 1.5 | 26.71 | 1.58 | 9.1 | 9.3743344 | 0.07525937 | |
| 2 | 27.13 | 2.32 | 12.1 | 11.437573 | 0.438809652 | |
| 3 | 25.12 | 3.68 | 15.3 | 13.864247 | 2.061386687 | |
| 4 | 22.11 | 4.82 | 15.9 | 15.147959 | 0.565565175 | |
| 6 | 16.53 | 6.41 | 17.1 | 16.36483 | 0.540475501 | |
| 8 | 12.26 | 7.28 | 16.8 | 16.854879 | 0.003011675 | |
| 10 | 9.08 | 7.64 | 17.2 | 17.033878 | 0.027596666 | |
| 12 | 6.73 | 7.66 | 16.9 | 17.043223 | 0.0205127 | |
| 16 | 3.69 | 7.11 | 16.8 | 16.763723 | 0.001316033 | |
| 20 | 2.03 | 6.19 | 17.2 | 16.22454 | 0.951522573 | |
| 24 | 1.11 | 5.21 | 16.2 | 15.494389 | 0.497887329 | |
| 30 | 0.45 | 3.87 | 15.3 | 14.113021 | 1.408919588 | |
| 36 | 0.18 | 2.81 | 13.2 | 12.469665 | 0.533389784 | |
| 42 | 0.07 | 2.01 | 11.1 | 10.670995 | 0.184044908 | |
| 48 | 0.03 | 1.43 | 8.9 | 8.8409215 | 0.003490267 | |
| | | | | | **8.43126913** | SS res |

**European Patent**

**Office**

**PARTIAL EUROPEAN SEARCH REPORT**   Application Number

which under Rule 45 of the European Patent Convention EP 96 10 5625
shall be considered, for the purposes of subsequent
proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| | No relevant documents disclosed ----- | | G06F17/00 A61K9/00 |

TECHNICAL FIELDS
SEARCHED     (Int.Cl.6)

A61K

## INCOMPLETE SEARCH

The Search Division considers that the present European patent application does not comply with
the provisions of the European Patent Convention to such an extent that it is not possible to carry
out a meaningful search into the state of the art on the basis of some of the claims
Claims searched completely :
Claims searched incompletely :
Claims not searched :
Reason for the limitation of the search:

see sheet C

| Place of search | Date of completion of the search | Examiner |
|---|---|---|
| THE HAGUE | 19 July 1996 | Benz, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C07)

EP 96105625.6
-C-


Meaningfull search not possible on the basis of all claims:
See Article 52(2) a) EPC.